# EUROPEAN PATENT SPECIFICATION

(11) **EP 0 731 782 B1**
(45) Date of publication and mention of the grant of the patent: **25.08.1999**
(21) Application number: 95923916.1
(22) Date of filing: 16.06.1995
(51) Int. Cl.: C07C 46/00, C07C 50/16

(54) **PROCESS FOR CATALYTICALLY DEHYDROGENATING ANTHRAHYDROQUINONE**
VERFAHREN ZUR KATALYTISCHEN DEHYDOSIERUNG VON ANTHRAHYDROCHINON
PROCEDE DE DESHYDROGENATION CATALYTIQUE D'ANTHRAHYDROQUINONE

(30) Priority: 29.09.1994 US 315174
(43) Date of publication of application: 18.09.1996
(73) Proprietor: MARATHON OIL COMPANY, Findlay, OH 45840 (US)
(72) Inventor: PLUMMER, Mark, A., Littleton, CO 80122 (US)
(74) Representative: Thomas, Roger Tamlyn
(86) International application number: US9507650
(87) International publication number: WO9610005

(56) References cited:
- WO-A-94/12431
- US-A- 4 592 905

## Description

The present invention relates to a process for catalytically dehydrogenating an anthrahydroquinone to the corresponding anthraquinone and hydrogen, and more particularly, to such a process wherein the catalyst electronegativity is selected to increase anthraquinone and hydrogen selectivity to substantially one hundred percent and thereby essentially eliminate unwanted hydrogenolysis.

In many processes, solutions containing an anthrahydroquinone are thermally or catalytically regenerated thereby producing the initial anthraquinone form which is usually recycled for use in a continuous process. One such process involves contacting within a reactor a feed gas containing hydrogen sulfide with an anthraquinone which is dissolved in a polar organic solvent. The resulting reaction between hydrogen sulfide and the anthraquinone yields sulfur and the corresponding anthrahydroquinone. The sulfur precipitates from the solution and is recovered as a product while the remaining solution containing anthrahydroquinone is thermally or catalytically regenerated producing the initial anthraquinone form and releasing hydrogen gas. The anthraquinone is recycled back to the reactor and hydrogen gas is recovered as a product.

Regeneration or dehydrogenation of an anthrahydroquinone often involves the use of supported metal catalysts. For example, metal oxide catalysts, e.g. chromium oxide, on alumina supports and platinum-ruthenium catalysts on alumina or silica-alumina supports have been employed in the catalytic dehydrogenation of anthrahydroquinones. However, the particular support employed or the metal oxide catalyst can cause hydrogenolysis during the catalytic dehydrogenation of anthrahydroquinones which results in the undesirable production of water and anthrones and/or anthranols. Thus, a need exists for such a process for the catalytic dehydrogenation of anthrahydroquinones wherein the anthrahydroquinone is entirely converted to anthraquinone and hydrogen products, and thus, unwanted hydrogenolysis by-products, such as anthrones and/or anthranols are eliminated.

US 4592905 discloses the conversion of hydrogen sulphide gas to sulfur and hydrogen by reacting the hydrogen sulphide with an anthraquinone which is dissolved in a polar hydrocarbon solvent. Insoluble sulfur, for example S₈ or other forms of polymeric sulfur, is withdrawn from the H₂S reactor as a precipitate in the reaction solution and is separated from solution by filtration, centrifugation or other means known in the art. After removal of the sulfur product, the withdrawn solution contains anthrahydroquinone solvent and any other unreactive compounds from the feed gas. Substantially all unreacted feed gas constituents, including H₂S and CO₂, are removed from the solution in a flash tank for recycle to the H₂S reactor. The solution from the flash tank is then fed to a dehydrogenation reactor where anthrahydroquinone is catalytically or thermally converted to anthraquinone and hydrogen gas. Catalytic dehydrogenation of the anthrahydroquinone is noted as being a function of catalyst type and dehydrogenation temperature. US 4592905 teaches that catalyst metals which are electron attracting or p-type conductors also minimize anthrone production and correspondingly maximize anthraquinone production.

The present invention aims to provide a process for catalytically dehydrogenating an anthrahydroquinone to the corresponding anthraquinone and hydrogen while eliminating substantial unwanted hydrogenolysis by-products.

The present invention further aims to provide a process for selecting a catalyst which results in complete selectivity to anthraquinone and hydrogen in the catalytic dehydrogenation of an anthrahydroquinone.

To achieve the foregoing and other aims, and in accordance with the purposes of the present invention, as embodied and broadly described herein, one characterization of the present invention is a process for dehydrogenating an anthrahydroquinone to form anthraquinone and hydrogen. The process comprises selecting a catalyst having an electronegativity greater than 2.30. The anthrahydroquinone which is dissolved in a polar organic solvent is reacted in the presence of the catalyst so as to substantially entirely convert anthrahydroquinone to anthraquinone and hydrogen.

In another characterization of the present invention, a process is provided for converting hydrogen sulfide gas to sulfur and hydrogen. The process comprises contacting the hydrogen sulfide gas with a polar organic solvent having an anthraquinone dissolved therein. The hydrogen sulfide gas reacts with the anthraquinone to product sulfur and an anthrahydroquinone in the solvent. The anthrahydroquinone is catalytically dehydrogenated to anthraquinone and hydrogen in the presence of a catalyst selected to have an electronegativity greater than 2.30 so as to increase the selectivity of converting anthrahydroquinone to anthraquinone and hydrogen to about 100%

The invention will now be described, by way of example only, with reference to the accompanying drawings, in which:-

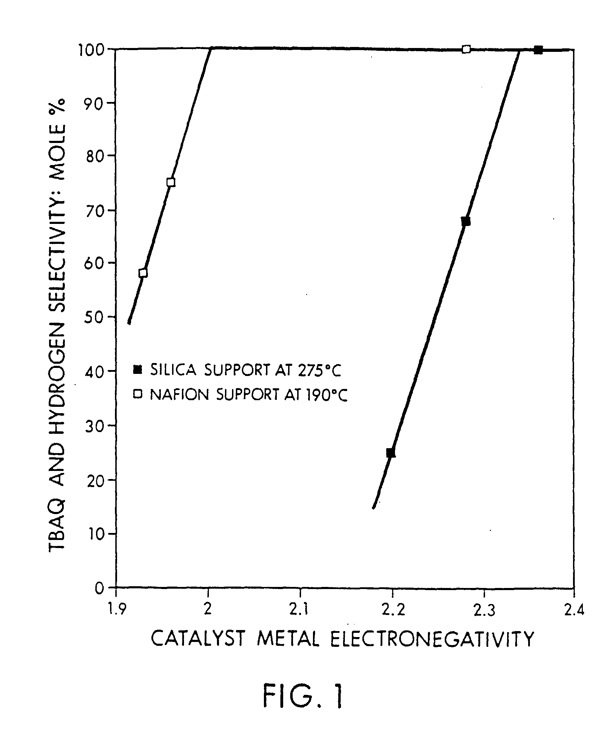

FIG. 1 is a graph which depicts hydrogen and t-butyl anthraquinone (TBAQ) production selectivity during the dehydrogenation of t-butyl anthrahydroquinone (H₂TBAQ) as a function of the catalyst metal electronegativity for two different metal supports with different electronegativities.

The present invention relates to the dehydrogenation of an anthrahydroquinone to the corresponding anthraquinone and hydrogen, as is employed in industrial processes, usually to regenerate anthraquinone for use in another process stage. For example, in one process the anthraquinone product from such dehydrogenation reaction which is dissolved in a polar organic solvent is used to contact a feed gas containing hydrogen sulfide (H₂S) in a reactor. Should the feed gas contain large quantities of gases other than hydrogen sulfide which are inert to this process, such as nitrogen, carbon dioxide, methane or other low molecular weight hydrocarbons, the feed gas and polar organic solvent containing an anthraquinone may be initially contacted in an absorber. The feed gas may contain other sulfur compounds, such as COS, CS₂ and mercaptans, which are converted in the process to H₂S, recycled to this reactor, and converted to sulfur. The solvent preferentially solubilizes hydrogen sulfide from the feed gas to form a reaction solution which is maintained in the reactor at a temperature of from about 0° C. to about 70° C. and at a H₂S partial pressure of from about 0.05 to about 4.0 atmospheres and for a time which is sufficient to convert the hydrogen sulfide and anthraquinone to sulfur and anthrahydroquinone.

The reaction solution is then removed from the H₂S reactor and the insoluble sulfur, e.g. S₈ or other forms of polymerized sulfur, is separated therefrom by filtration, centrifugation, or any other means known in the art. The remaining reaction solution, comprising the polar organic solvent, dissolved anthrahydroquinone, and any unreacted anthraquinone, is then heated to from about 100° C. to about 150° C. at atmospheric pressure and fed to a flash tank where substantially all unreacted feed gas constituents, including H₂S and CO₂ are removed from solution and recycled to the reactor. The solution is withdrawn from the flash tank and may be further heated to from about 150° C. to about 275° C. at a pressure sufficient to prevent solvent boiling. The heated solution is then fed to a dehydrogenation reactor where the anthrahydroquinone is catalytically converted to anthraquinone and hydrogen gas (H₂) under the temperature and pressure conditions stated above.

In accordance with the present invention, Applicant has discovered that the selection of a catalyst having an electronegativity of greater than 2.30 and preferably greater than 2.35 unexpectedly results in hydrogen and anthraquinone product selectivity of substantially one hundred percent in the dehydrogenation of anthrahydroquinones, and thus, substantially eliminates unwanted hydrogenolysis by-products, such as anthrones and/or anthranols. The catalyst may be a solid or a liquid, and preferably is heterogenous or immisicible with the polar organic solvent at the dehydrogenation raction conditions. The catalyst may be a metal element, a metal compound, or such metal element and/or metal compound dispersed upon a solid support or the catalyst may be a liquid compound. Thus, in accordance with the present invention, the entire catalyst or a component of the catalyst is selected to have an electronegativity of greater than 2.30. If the catalyst is a metal element a metal compound or a liquid compound, then such metal element, metal compound or liquid compound should have an electronegativity greater than 2.30. If the catalyst is a metal element or metal compound dispersed on a support, then either the support or the metal element or compound should have an electronegativity greater than 2.30. Where the support has an electronegativity greater than 2.30, then the metal element or the metal compound dispersed thereon should have an electronegativity greater than 2.00. As utilized throughout this specification, the term "electronegativity" refers to the Pauling electronegativity as discussed at page 215, volume 17 (1961) of the "Journal Inorganic Nuclear Chemistry" by A.L. Allred. Electronegativities of several metal compounds and function groups are detailed in Structure and Bonding 66 Electronegativity, Editors K.D. Sen and C.K. Jorgensen, Springer-Verlag, Berlin Heidelberg, 1987. While it is not exactly understood why the use of a catalyst electronegativity of greater than 2.30 results in substantially complete conversion of anthrahydroquinone to anthraquinone and hydrogen product during dehydrogentation, it is believed that the need to remove an electron pair from the anthrahydroquinone during the conversion to anthraquinone and hydrogen is accomplished by increasing catalyst electronegativity.

Suitable metal elements for use in accordance with the present invention are, for example, gold, selenium, tungsten, and mixtures thereof. These metals can be used as a catalyst in the process of the present invention or can be dispersed on supports which includes those supports having an electronegativity less than 2.30, such as silica, alumina, and mixtures thereof. Alternatively, these metals catalysts or other metal catalysts having electronegativities greater than 2.00, for example platinum (Pt), palladium (Pd), rhodium (Rh), molybdenum (Mo), titanium (Ti), rhenium (Re), or mixtures thereof, can be dispersed on supports which have electronegativities greater than 2.30, for example a polyperfluorosulfonic acid polymer, such as a Nafion® polymer which is commercially available from E. I. Du Pont de Memours & Co. Other suitable supports with electronegativities greater than 2.30 are carbon and hydrocarbons containing at least one of the group consisting of -BeF, -BF₂, -NF₂, -OF, -N(C₃H₇)₂, -OCH₃, -COOH, -OSO₃H, -CN, -NCS and -NCO. Also, metal compounds which have electronegativities greater than 2.30 can be used as a catalyst in accordance with the present invention, for example, WS₂, SeO, and metals attached to at least one of the group consisting of -BeF, BF₂, -NF₂, -OF, -N(C₃H₇)₂, -OCH₃, -COOH, -OSO₃H, -CN, -NCS and -NCO. Suitable liquid compounds for use in accordance with the present invention are perfluoronated hydrocarbons, for example perfluorononane.

After the dehydrogenation reaction, anthraquinone in its initial form is withdrawh from the dehydrogenation reactor dissolved in the polar organic solvent and is recycled to the H₂S reactor, while the H₂ gas is recovered as a commercial product. Preferably, the process of the present invention is operated as a continuous process.

Suitable polar organic solvents for use in such process include N-methyl-2-pyrrolidinone, N,N-dimethylacetamide, N,N-dimethylformamaide, sulfolane (tetrahydrothiophene-1,1-dioxide), acetonitrile, 2-nitropropane, propylene carbonate and mixtures thereof. The most preferred solvent is N-methyl-2-pyrrolidinone (NMP). Useful anthraquinones are ethyl, t-butyl, t-amyl and s-amyl anthraquinones and mixtures thereof because of their relatively high solubilities in most polar organic solvents.

The following examples demonstrate the practice and utility of the present invention, but are not to be construed as limiting the scope thereof.

### EXAMPLE 1

A mixture containing 18 wt% t-butyl anthrahydroquinone (H₂TBAQ) and 7 wt% t-butyl anthraquinone (TBAQ) in n-methyl-2-pyrrolidinone (NMP) is dehydrogenated at 275° C and 72 psia using three catalysts in three separate runs. One catalyst composition is 2.75 wt% platinum (Pt) metal dispersed on a silica support (Grace Grade 57), the second catalyst composition is 2.75 wt% palladium (Pd) metal on a silica support (Grace Grade 57), and the third catalyst composition is 2.75 wt% tungsten (W) metal on the same support. The electronegativities of the Pd, Pt and W metals are 2.20, 2.28, and 2.36, respectively. The electronegativity of the support is estimated to be significantly below 2.30.

The selectivity of converting the H₂TBAQ to hydrogen and TBAQ is 68 mole% using the Pt catalyst, 25 mole% using the Pd catalyst, and 100% using the W catalyst. In each instance, the balance of the H₂TBAQ is converted to the corresponding anthrone or anthranol and water.

These results, as correlated to the electronegativity of the metal catalysts, are illustrated in FIG. 1, and demonstrate that a metal electronegativity greater than 2.30 is sufficient to yield a selectivity to TBAQ and hydrogen of 100 mole % without production of unwanted anthrone or anthranol and water.

### EXAMPLE 2

A mixture of H₂TBAQ and TBAQ which is identical to that used in Example 1 is dehydrogenated using a Nafion® polymer from E.I. Du Pont de Memours & Co. as the support to which 0.8 meq of Pt/gm of Nafion® polymer have been ion exchanged with the hydrogen proton of the sulfonic acid group. The electronegativity of this support is between 2.30 (the electronegativity of the -CF₂- polymer backbone) and 2.91 (the electronegativity of the -CF₃ polymer terminal groups. The sulfonic acid group in this polymer has an electronegativity of 5.06. For comparison, a separate dehydrogenation run is conducted using Pt catalyst of Example 1. Dehydrogenation conditions are 190° C and 12 psia.

The selectivity of converting H₂TBAQ to TBAQ and hydrogen is 100 mole% for the Pt-Nafion® polymer catalyst and 7.7 mole% for the Pt-silica supported catalyst. This result illustrates the desirability of utilizing a catalyst support having an electronegativity of greater than 2.30 in the process of the present invention.

### EXAMPLE 3

An mixture of H₂TBAQ and TBAQ which is identical to that used in Example 1 is dehydrogenated in separate runs at 190° C and 12 psia using a Nafion® polymer from E.I. Du Pont de Memours & Co. as the support to which either 0.8 meq of Ag/gm or Sn/gm of Nafion® polymer have been ion exchanged with the hydrogen proton of the sulfonic acid group. The electronegativity of Ag and Sn are 1.93 and 1.96, respectively. The electronegativity of this support is between 2.30 (the electronegativity of the -CF₂- polymer backbone) and 2.91 (the electronegativity of the -CF₃ polymer terminal group. The sulfonic acid group in this polymer has an electronegativity of 5.06. The selectivity of converting the H₂TBAQ to hydrogen and TBAQ is 59 mole% for the Ag catalyst and 75 mole% for the Sn catalyst, the balance of the H₂TBAQ is converted to the corresponding anthrone or anthranol and water. This result is plotted versus metal electronegativity in FIG. 1. The results indicate that if a support with an electronegativity of greater than 2.30 is used , the dispersed metal electronegativity needs to be greater than 2.00.

### EXAMPLE 4

A liquid mixture containing 11.8 wt% t-butyl anthrahydroquinone (H₂TBAQ) and 13.2 wt% t-butyl anthraquinone (TBAQ) in n-methyl-2-pyrolidinone (NMP) is dehydrogenated by contacting the mixture by means of intense mechanical mixing with liquid perfluorononane at 150°C to 180°C for 30 minutes. The NMP mixture and perfluorononane are immiscible liquids at these conditions. Upon conclusion of this dehydrogenation reaction, the H₂TBAQ content in the NMP mixture is reduced to 0 wt% and the only products produced are TBAQ and hydrogen. Accordingly, undesirable by-products of hydrogenolysis, i.e. t-butyl anthrones (TBAN) and t-butyl anthranols (TBAL), are not produced. The two immiscible liquid phases, i.e. the NMP reaction mixture and perfluorononane, are then separated at room temperature and pressure. The electronegativity of perfluorononane is between 2.30 which is the electronegativity of the -CF₂- backbone and 2.91 which is the electronegativity of the -CF₃ terminal group.

### EXAMPLE 5

Two 25 gm liquid samples containing 11.64 wt% H₂TBAQ and 12.74 wt% TBAQ in NMP solvent are placed into two separate reactors. 2.5 gms of liquid perfluorononane catalyst is also added to each of the two liquid samples in each reactor. The vapor space above the two immiscible liquids in each reactor is purged with hydrogen to remove all air. One reactor is placed in a hot oil bath at 150°C and the immiscible liquids are gently mixed for 10 minutes to contact the perfluorononane with the NMP solution. The pressure in the reactor increased from 0 to a stable value of 29 psig indicating the evolution of hydrogen. The reactor contents are then cooled to room temperature and the NMP solution is analyzed. The amount of H₂TBAQ in the solution decreases to 8.80 wt% while the amount of TBAQ increases to 15.58 wt%. No TBAN or TBAL by-products are produce indicating that hydrogenolysis does not occur.

The second reactor is placed in a hot oil bath at 200°C and the immiscible liquids are gently mixed for 16 minutes to contact the perfluorononane with the NMP solution. In the first 5 minutes of mixing,i.e. reaction, the pressure in the second reactor increased from 0 to 45 psig at which point the produced hydrogen is removed from the second reactor until a reactor pressure of 15 psig is reached. In a similar manner, the reactor pressure is reduced from 45 to 15 psig after 7 minutes of total reaction time and from 31 to 15 psig after 13 minutes of total reaction time. After 16 minutes, the reactor pressure increases to a stable pressure of 26 psig and the reaction was quenched at room temperature. The NMP solution is analyzed and determined to contain 5.34 wt% H₂TBAQ and 19.04 wt% TBAQ. No TBAN or TBAL by-products are produce indicating that hydrogenolysis does not occur.

Perfluorononane is easily removed from the NMP solution emanating from each reactor by conventional liquid phase separation techniques.

Although selection of a catalyst having an electronegativity of greater than 2.30 has been described above with respect to a process for removing hydrogen sulfide from a feed gas and recovering sulfur and hydrogen as products, the process of the present invention can be applied to any process where an anthrahydroquinone undergoes a dehydrogenation reaction to regenerate anthraquinone for further use. For example, a process for the conversion of hydrogen chloride gas to hydrogen and chlorine gases.

## Claims

1. A process tor dehydrogenating an anthrahydroquinone to form anthraquinone and hydrogen, said process comprising:
selecting a catalyst having an electronegativity greater than 2.30; and
dehydrogenating an anthrahydroquinone which is dissolved in a polar organic solvent in the presence of said catalyst thereby substantially entirely converting said anthrahydroquinone to anthraquinone and hydrogen.

2. The process of claim 1 wherein said catalyst is a solid.

3. The process of claim 2 wherein said catalyst is a metal element, a metal compound, or a metal element or a metal compound dispersed on a support.

4. The process of claim 3 wherein said metal element is gold, selenium, tungsten, or mixtures thereof.

5. The process of claim 3 wherein said metal compound is WS₂, SeO, or metals attached to at least one of the group consisting of -BeF, -BF₂, -NF₂, -OF, -N(C₃H₇)₂, -OCH₃, -COOH, -OSO₃H, -CN, -NCS and -NCO.

6. The process of claim 3 wherein said support has an electronegativity less than 2.30.

7. The process of claim 6 wherein said support is silica, alumina, or mixtures thereof.

8. The process of claim 7 wherein the metal element or metal compound dispersed on said support has an electronegativity greater than 2.30.

9. The process of claim 3 wherein said support has an electronegativity greater than 2.30.

10. The process of claim 9 wherein said support is a polyperfluorosulfonic acid polymer, carbon, or hydrocarbons containing at least one of the group consisting of -BeF, -BF₂, -NF₂, -OF, -N(C₃H₇)₂, -OCH₃, -COOH, -OSO₃H, -CN, -NCS and -NCO.

11. The process of claim 10 wherein the metal element or metal compound dispersed on said support has an electronegativity greater than 2.00.

12. The process of claim 1 wherein said catalyst is a liquid.

13. The process of claim 12 wherein said catalyst is a perfluorononated hydrocarbon.

14. The process of claim 13 wherein said catalyst is perfluorononane.

## Patentansprüche

1. Verfahren zum Dehydrieren eines Anthrahydrochinons unter Bildung von Anthrachinon und Wasserstoff, wobei das Verfahren umfaßt:
Auswählen eines Katalysators mit einer Elektronegativität von mehr als 2,30; und
Dehydrieren eines Anthrahydrochinons, das in einem polaren organischen Lösungsmittel gelöst ist, in Gegenwart des Katalysators, wodurch im wesentlichen das gesamte Anthrahydrochinon in Anthrachinon und Wasserstoff umgewandelt wird.

2. Verfahren gemäß Anspruch 1, wobei der Katalysator ein Feststoff ist.

3. Verfahren gemäß Anspruch 2, wobei der Katalysator ein elementares Metall, eine Metallverbindung oder ein elementares Metall oder eine Metallverbindung, das bzw. die auf einem Träger dispergiert ist, ist.

4. Verfahren gemäß Anspruch 3, wobei es sich bei dem elementaren Metall um Gold, Selen, Wolfram oder Gemische davon handelt.

5. Verfahren gemäß Anspruch 3, wobei es sich bei der Metallverbindung um WS₂, SeO oder um Metalle, die an wenigstens einen Rest gebunden sind, der aus der Gruppe ausgewählt ist, die aus -BeF, -BF₂, -NF₂, -OF, -N(C₃H₇)₂, -OCH₃, -COOH, -OSO₃H, -CN, -NCS und -NCO besteht, handelt.

6. Verfahren gemäß Anspruch 3, wobei der Träger eine Elektronegativität von weniger als 2,30 hat.

7. Verfahren gemäß Anspruch 6, wobei es sich bei dem Träger um Siliciumoxid, Aluminiumoxid oder Gemische davon handelt.

8. Verfahren gemäß Anspruch 7, wobei das elementare Metall oder die Metallverbindung, das bzw. die auf dem Träger dispergiert ist, eine Elektronegativität von mehr als 2,30 hat.

9. Verfahren gemäß Anspruch 3, wobei der Träger eine Elektronegativität von mehr als 2,30 hat.

10. Verfahren gemäß Anspruch 3, wobei es sich bei dem Träger um ein Polyperfluorsulfonsäure-Polymer, Kohlenstoff oder Kohlenwasserstoffe, die wenigstens einen Rest enthalten, der aus der Gruppe ausgewählt ist, die aus -BeF, -BF₂, -NF₂, -OF, -N(C₃H₇)₂, -OCH₃, -COOH, -OSO₃H, -CN, -NCS und -NCO besteht, handelt.

11. Verfahren gemäß Anspruch 10, wobei das elementare Metall oder die Metallverbindung, das bzw. die auf dem Träger dispergiert ist, eine Elektronegativität von mehr als 2,00 hat.

12. Verfahren gemäß Anspruch 1, wobei der Katalysator eine Flüssigkeit ist.

13. Verfahren gemäß Anspruch 12, wobei der Katalysator ein perfluornonanhaltiger Kohlenwasserstoff ist.

14. Verfahren gemäß Anspruch 13, wobei es sich bei dem Katalysator um Perfluornonan handelt.

## Revendications

1. Un procédé pour déshydrogéner une anthrahydroquinone afin de former une anthraquinone et de l'hydrogène, ledit procédé comprenant :
la sélection d'un catalyseur présentant une électronégativité supérieure à 2,30 ; et
la déshydrogénation d'une anthrahydroquinone qui est dissoute dans un solvant organique polaire en présence dudit catalyseur en convertissant ainsi de manière pratiquement complète ladite anthrahydroquinone en anthraquinone et en hydrogène.

2. Le procédé de la revendication 1 dans lequel ledit catalyseur est un solide.

3. Le procédé de la revendication 2 dans lequel ledit catalyseur est un élément métallique, un composé métallique ou encore un élément métallique ou un composé métallique dispersé sur un support.

4. Le procédé de la revendication 3 dans lequel ledit élément métallique est l'or, le sélénium, le tungstène, ou leurs mélanges.

5. Le procédé de la revendication 3 dans lequel ledit composé métallique est WS2, SeO, ou des métaux fixés à au moins un du groupe se composant de -BeF, BF₂, -NF₂, -OF, -N(C₃H₇)₂, -OCH₃, -COOH, -OSO₃H, -CN, -NCS et -NCO.

6. Le procédé de la revendication 3 dans lequel ledit support présente une électronégativité inférieure à 2,30.

7. Le procédé de la revendication 6 dans lequel ledit support est la silice, l'alumine, ou leurs mélanges.

8. Le procédé de la revendication 7 dans lequel l'élément métallique ou le composé métallique dispersé sur ledit support présente une électronégativité supérieure à 2,30.

9. Le procédé de la revendication 3 dans lequel ledit support présente une électronégativité supérieure à 2,30.

10. Le procédé de la revendication 9 dans lequel ledit support est un polymère de l'acide polyperfluorosulfonique, du carbone, ou des hydrocarbures contenant au moins un du groupe se composant de -BeF, BF₂, -NF₂, -OF, -N(C₃H₇)₂, -OCH₃, -COOH, -OSO₃H, -CN, -NCS et -NCO.

11. Le procédé de la revendication 10 dans lequel l'élément métallique ou le composé métallique dispersé sur ledit support présente une électronégativité supérieure à 2,00.

12. Le procédé de la revendication 1 dans lequel ledit catalyseur est un liquide.

13. Le procédé de la revendication 12 dans lequel ledit catalyseur est un hydrocarbure perfluorononé.

14. Le procédé de la revendication 13 dans lequel ledit catalyseur est du perfluorononane.
